# EUROPEAN PATENT SPECIFICATION

(11) **EP 2 315 828 B1**
(45) Date of publication and mention of the grant of the patent: **01.07.2015**
(21) Application number: 09784152.2
(22) Date of filing: 13.07.2009
(51) Int. Cl.: C12N 5/0735, C07K 14/47

(54) **Method for culturing induced pluripotent stem cells with a lectin**
Verfahren zur Kultivierung von induzierten pluripotenten Stammzellen mit einem Lektin
Procédé de culture de cellules souches pluripotentes induites avec une lectine

(30) Priority: 11.07.2008 FI 20085724; 11.07.2008 US 171866
(43) Date of publication of application: 04.05.2011
(73) Proprietor: Glykos Finland Oy, 00790 Helsinki (FI)
(72) Inventor: IMPOLA, Ulla, FI-00530 Helsinki (FI); TIITTANEN, Minna, FI-00310 Helsinki (FI); MIKKOLA, Milla, FI-00640 Helsinki (FI); PARTANEN, Jukka, FI-00760 Helsinki (FI); NATUNEN, Jari, FI-01520 Vantaa (FI); SATOMAA, Tero, FI-00700 Helsinki (FI); SAARINEN, Juhani, FI-00370 Helsinki (FI)
(74) Representative: Aalto, Juha-Matti
(86) International application number: PCT/FI2009/050624
(87) International publication number: WO 2010/004096

(56) References cited:
- EP-A- 1 674 566
- WO-A-2007/054620
- WO-A-2007/066352
- WO-A-2008/000918
- WO-A1-2008/087257
- WO-A2-2007/006870
- US-A1- 2004 121 301
- US-A1- 2005 060 779
- VAS VIRAG ET AL: "Biphasic effect of recombinant galectin-1 on the growth and death of early hematopoietic cells" STEM CELLS (MIAMISBURG), vol. 23, no. 2, February 2005 (2005-02), pages 279-287, XP002563291 ISSN: 1066-5099
- TAKASHI MURAMATSU ET AL: "Carbohydrate antigens expressed on stem cells and early embryonic cells" GLYCOCONJUGATE JOURNAL, KLUWER ACADEMIC PUBLISHERS, BO, vol. 21, no. 1-2, 1 January 2004 (2004-01-01), pages 41-45, XP019206995 ISSN: 1573-4986

## Description

### Field of the specification

The present specification relates to a method for culturing human embryonic stem cells (hESCs) and/or induced pluripotent stem (iPS) cells on a lectin. The specification relates also to the use of a lectin in a method for culturing human embryonic stem cells (hESCs) and/or induced pluripotent stem (iPS) cells and a culture medium composition containing a lectin attached on the culturing plates.

### Background of the invention

Traditional methods for culturing human embryonic stem cells (hESCs) require the direct use of mouse embryonic fibroblasts (MEFs) as a feeder layer, or feeder-conditioned medium or serum. A medium for a feeder-free culture of hESCs includes an extracellular matrix extracted from a mouse sarcoma and is sold under the trademark Matrigel™ (BD Bioscience, US). Matrigel™ is mostly comprised of laminin and collagen and these compounds in purified form have also been tried in culturing hESCs.

Matrigel™ and the other feeder-free media used currently in cultures suffer from xeno contamination, and in addition are subject to large variability caused by containing growth factors and other undefined molecules.

Mallon B.S. et al. have reviewed the attempts made toward xenofree cuture of hESCs in The International Journal of Biochemistry and Cell Biology 38, 1063-1075, 2006. As can be concluded, the culture of hESCs suffers with respect to both technical and clinical potential by the use of cells or extracts originating from animal sources, such as mouse embryonic fibroblasts and an extract from a mouse sarcoma. The current culture methods are also laborious and difficult to scale. Further, it is often hard to maintain the cells in uniform quality and in an undifferentiated form.

One of the biggest problems of the current methods and media for culturing hESCs and embryonic stem cell-like cells, such as iPS cells, arises from the use of animal-derived material in the cultture medium.

This problem has now been solved in accordance of the present specification by providing a method for culturing iPS cells using a medium containing a lectin as a culturing matrix.

### Brief description of the invention

The invention is defined by the claims.

### Brief description of the drawings

The following drawings are not meant to limit the scope of the invention as defined in the claims in any way.
Figure 1 shows the colonies of (A); FES 29 cells cultured on ECA-lectin for 6 passages (original magnification 4 x), (B); FES 29 cells during passage 14 on ECA (magnification 10 x) and (C); FES 30 cells cultured on ECA for 7 passages (magnification 10 x) obtained in Example 1.
Figure 2 shows the FACS analysis of the surface markers (A); SSEA3 and (B) Tra-1-60 (or Tra-1-81) expressions on FES 29 cells during ECA culture from the beginning of ECA culture (passage 0) to passage 8, and (C) SSEA3 and (D) Tra-1-60 (or Tra-1-81) expressions on FES 30 cells during ECA culture from the beginning of ECA culture (passage 0) to passage 8 obtained in Example 1. The surface marker expressions in the control Matrigel cultures are shown for comparison (p=passage ECA/Matrigel).
Figure 3 shows the FES 29 cells cultured in suspension for EB formation after 9 passages on ECA described in Example 1.
Figure 4 shows the hESC colonies on ECA in StemPro^{®} medium obtained in Example 2: (A) FES 29 cells cultured on ECA for 9 passages: first 7 passages in conditioned medium and then 2 passages in StemPro^{®} definitive medium and (B) FES 29 cells during passage 3 in StemPro^{®} medium on ECA passage 10.
Figure 5 shows the FACS-analysis of the expression of the two surface markers SSEA-3 and Tra-1-60 of undifferentiated hESCs described in Example 2. FES29-cells were cultured on ECA for 10 passages and with StemPro^{®}-medium for the last 3 passages.
Figure 6 shows the EBs formed from FES 29 cells after 12 ECA passages and 4 StemPro^{®} passages obtained in Example 2.
Figure 7 shows (A) FiPS1-5 and (B-C) FiPS6-12 cell colonies after 5 passages on ECA-lectin in conditioned medium obtained in Example 3.
Figure 8 shows EBs were formed from FiPS6-12 cells after 6 passages on ECA obtained in Example 3.
Figure 9 shows a list of lectins whose amino acid sequences are highly homologous to that of ECA. Potential N-glycosylation sites have been indicated with highlighting. Lysine residue, which can be used to link the lectin to a surface, have been shown in bolded italics.
Figure 10 shows surface expression of two markers for stem cellness, Tra-1-60 and SSEA-3, on the FiPS 6-14 cells as determined by standard FACS analysis. The expression of Tra-1-60 and SSEA-3 on Fi PS 6-14 cells during ECA culture from the beginning of ECA culture (passage 0) to passage 11 (#p21, figure 10A) and to passage 12 (#p18, figure 10B) are shown. The surface marker expressions in the #p21 control Matrigel cultures are shown for comparison. p = passage.

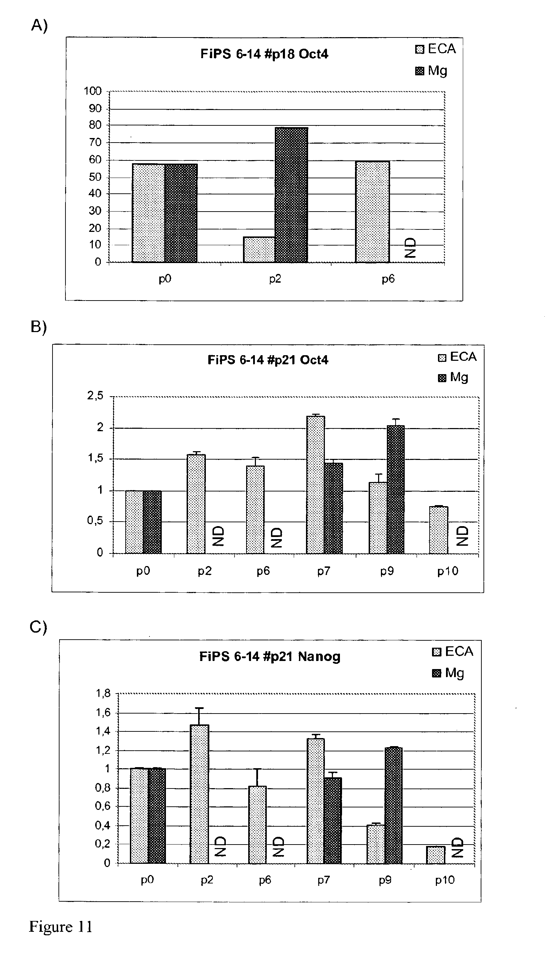

Figure 11 shows mRNA expression levels of Oct-4 (both FiPS 6-14#p18 and FiPS 6-14#p21) and Nanog (FiPS 6-14#p21 only). The fold change in the expression levels of the genes were calculated with 2 -ΔΔCT method (Livak and Schmittgen, Methods 25, 2001). (A) Fold change of target gene (Oct-4 and Nanog) was calculated in relation to the expression of TBP endogenous control. Each value represents mean value of fold change (± S.E.) in gene expression relative to TBP. (B,C) Fold change in target gene was normalized to TBP endogenous control and to expression level at start of the growth (p0). Each value represents a mean ± S.D. of duplicate or triplicate sample. Results from one of two separate runs are shown. p = passage.
Figure 12 shows mRNA expression levels of stem cell markers (A) OCT-4 and (B) Nanog. The fold change of target gene (Oct-4 and Nanog) was calculated in relation to the expression of TBP endogenous control with 2 -ΔΔCT method (Livak and Schmittgen, Methods 25, 2001). Each value represents mean value of fold change (± S.E.) in gene expression relative to TBP. p = passage.
Figure 13 shows the expression levels of Oct-4 mRNA in FES 29 cells as relative units (RU; 2^{-ΔΔCt}) in which the threshold cycle (Ct) value of the marker gene was normalized to the Ct value of the housekeeping gene (Cyclophilin G; ΔCt). The differences were further compared to the starting point sample (ΔΔCt) and a calculation of 2^{-ΔΔCt} gave the relative value compared to the starting point (starting point sample gains value 1). p = passage.
Figure 14 shows photos of teratoma formed after transplantation of the FES 29 cells into nude mouse testis. Immunohistological examination showed tumour contained tissues representing all three germ layers, endoderm, mesoderm and ectoderm, as marked by arrows.

### Detailed description of the invention

The invention is defined by the claims.

Human embryonic stem cells (hESCs) are derived from the inner cell mass of 3-5 day-old blastocysts. hESCs pose telomerase activity and express surface markers SSEA-3, SSEA-4, Tra-1-60 and Tra-1-81. They proliferate on continuous basis when maintained in an appropriate culture environment and differentiate both in vivo and in vitro into endo-, meso- and ectoderm. The differentiation is detected by formation of embryoid bodies in vitro and teratoma in vivo. hESCs are considered to be the building blocks for all types of cells in humans and thus have huge potential in applications of cell therapy and regenerative medicine. In technologies for harvesting hESCs the embryo is either destroyed or not, i.e. it remains alive. In one embodiment of the specification, the hESCs are harvested by a method that does not include the destruction of a human embryo. With regard to the safety of the transplantation applications of hESCs and the derivatives thereof, it is important to reduce or even eliminate the xenogenic contamination of these cells.

Induced pluripotent stem (iPS) cells are a type of pluripotent stem cell derived from principally any non-pluripotent or differentiated cell, such as an adult somatic cell, that has been induced to have all essential features of embryonic stem cells (ESC). The techniques were first described in human cells by Takahashi et al. in Cell 131: 861-872, 2007. They demonstrated the generation of iPS cells from adult dermal fibroblasts by transduction of four transcription factors: Oct3/4, Sox2, Klf4 and c-Myc. Later, it has been shown that there are also other ways to generate similar cells (Lowry and Plath, Nature Biotechnology 26(11): 1246-1248, 2008) and basically, in addition to fibroblasts, any cell, such as a blood cell, derived from an embryo, a newborn, a child, an grown-up and/or an adult may be converted to an iPS cell line. The iPS cells are considered to be identical to natural pluripotent stem cells, such as embryonic stem cells, in many respects. The iPS cells hold enormous promise as this way, cells having all features of an ESC can be produced without ethical problems. Also, ESC-like cells can be readily produced from affected individuals to study molecular mechanisms of diseases and to test therapeutic molecules. As for ESC lines, the culturing of iPS cells has turned out to be demanding and there is great need to more established and better defined culture conditions. As glycosylation and glycan-mediated interactions are cell-type specific, prior art from other cells does not teach the glycobiology of iPS cells. Thus, effects of lectins on growth of iPS cells are not predictable and/or obvious.

In one embodiment of the specification, the method, composition and use are directed to culturing hESCs. In another embodiment of the specification, the method, composition and use are directed to culturing iPS cells.

In a further embodiment of the specification, the method, composition and use are directed to culturing hESCs selected from cell lines FES 22, FES 29 and/or FES 30. In a still further embodiment of the specification, the method, composition and use are directed to culturing iPS cells selected from cell lines FiPS1-5, FiPS5-7, FiPS6-12 and/or FiPS6-14.

Lectins are sugar-binding proteins. They typically play a role in biological recognition phenomena involving cells and proteins. Most of the lectins are basically non-enzymatic in action and non-immune in origin. Lectins occur ubiquitously in nature. They may bind to a soluble carbohydrate or to a carbohydrate moiety which is a part of a more complex carbohydrate structure, such as a glycoprotein or glycolipid. They typically agglutinate certain animal cells and precipitate glycoconjugates. Lectins serve many different biological functions from the regulation of cell adhesion to glycoprotein synthesis and the control of protein levels in the blood. Lectins are also known to play important roles in the immune system by recognizing carbohydrates that are found exclusively on pathogens or that are inaccessible on host cells. Lectins could be derived from plants, such as legume plants like beans, grains and seeds. In addition, lectins having an animal origin are known. Legume lectins are one of the largest lectin families with more than 70 lectin family members.

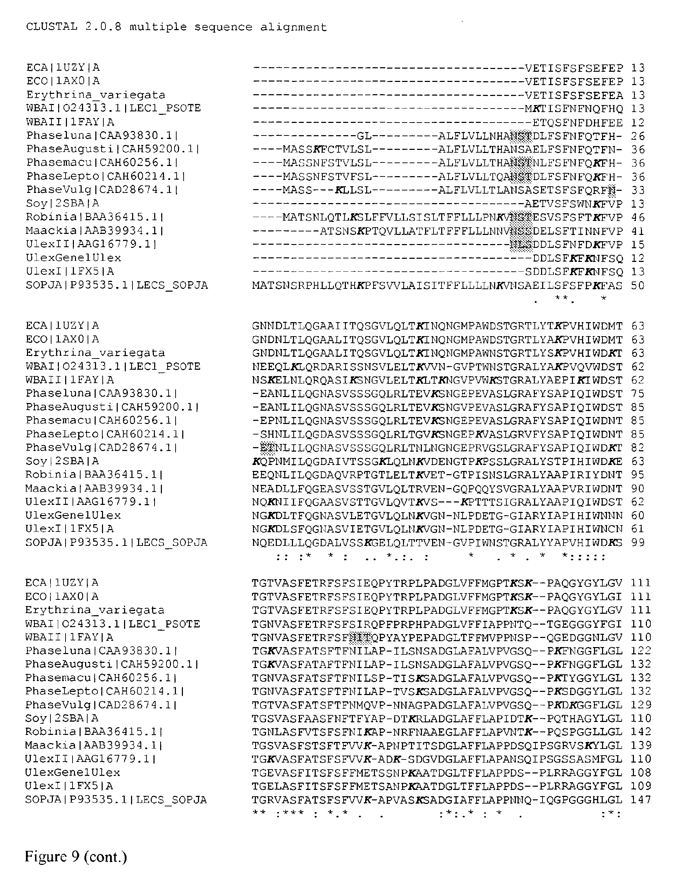

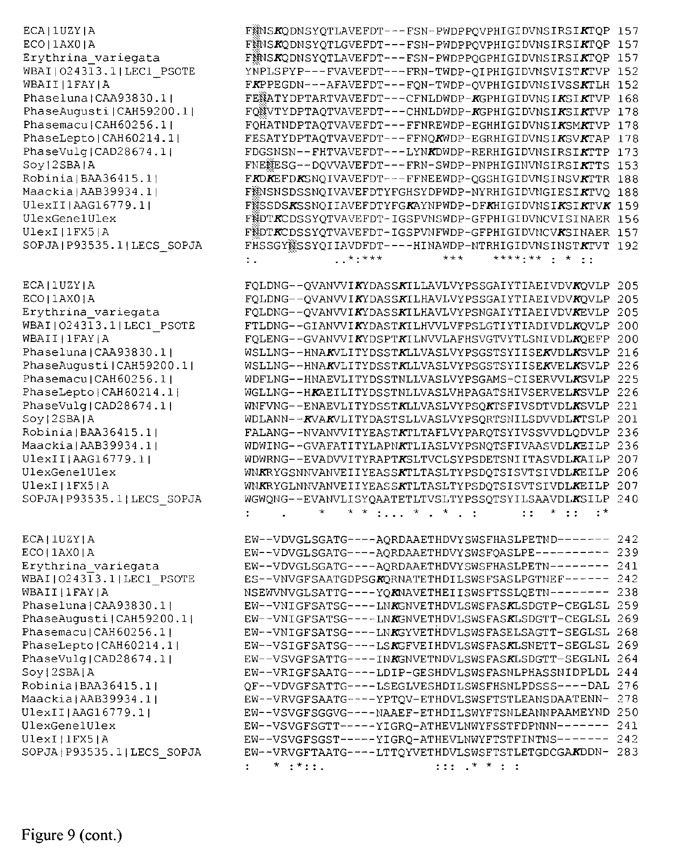

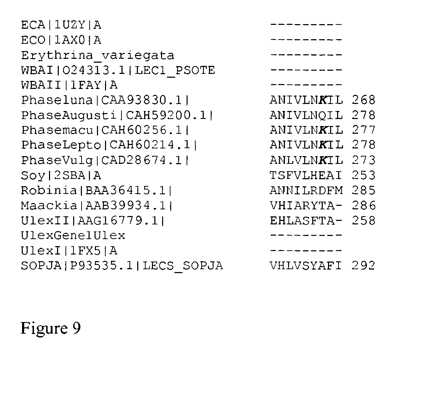

Known lectins isolated from plants are, for example, Con A, LCA, PSA, PCA, GNA, HPA, WGA, PWM, TPA, ECA, DSA, UEA-1, PNA, SNA and MAA. Galectins are a family of lectins having mammalian origin. Lectins recognizing the "terminal N-acetyllactosamine" structure (Fucα2)ₙGalβ4GlcNAc, wherein n is 0 or 1, are a group of preferred lectins of the present specification. Lectins recognizing both the "terminal N-acetyllactosamine" structures wherein n is 0 and n is 1, is another preferred group of lectins. Optionally, the lectins do not essentially recognize sialylated and/or sulphated Galβ4GlcNAc-structures. These lectins include, in particular, ECA (*Erythrina cristagalli* lectin), DSA (*Datura Stramonium* lectin) and UEA-1 (Ulex europeaus agglutinin-I), as well as galectin lectins. In addition, a number of other natural lectins may have the specificity of recognizing and/or binding to the "terminal N-acetyllactosamine" structure. Furthermore, natural lectins can be mutagenized to improve their binding or to obtain binding specificity to the "terminal N-acetyllactosamine". These lectins recognize and/or bind to the "terminal N-acetyllactosamine" structure in an amount and/or extent adequate to fulfil the function for supporting the growth of the cell. A list of lectins, whose amino acid sequences are highly homologous to ECA is shown in Figure 9. These lectins potentially have or may readily be biotechnologically modified by e.g. mutagenesis to have the same activity as ECA. ECA lectin refers also to variants of ECA that are modified in amino acid positions defined in Figure 9, optionally with the amino acid variations shown in Figure 9.

In one embodiment of the specification, the lectin is selected from ECA, UEA-1, galectin lectins and/or an essentially similar protein biotechnologically produced thereof. In another embodiment of the specification, the lectin is selected from lectins having characteristics substantially similar to ECA, UEA-1 and/or galectin lectins with regard to supporting the growth of the hESCs and/or iPS cells.

In one embodiment of the specification, the lectin is an animal-free galectin, that is, a recombinant lectin protein produced in cell culture system, preferably in a non-animal cell culture system.

In one embodiment of the specification, lectins include also oligosaccharide-binding protein domains and peptides derived from lectins. Preferably the lectins do not contain a non-lectin domain, such as an enzyme domain or toxic domain found, for example, in ricin agglutinin (RCA). The lectins of the present specification further include any polypeptide or equivalent being functionally a lectin. Antibodies and oligosaccharide-binding enzymes are examples of the proteins being functional lectins. Prefererred enzymes include fucosidases and galactosidases modified to remove the catalytic activity. The antibodies include all types of natural and genetically engineered variants of immunoglobulin proteins. Preferred antibodies include blood group H type II and terminal N-acetylactosamine binding antibodies.

In the present specification the term "terminal N-acetyllactosamine" refers to a neutral N-acetyllactosamine with a non-reducing terminal end; the neutral means that the structure is not modified by sialic acid or other acidic residues. Preferably terminal N-acetyllactosamine is non-substituted type II N-acetyllactosamine or its α2'-fucosylated variant structure (H-type II structure) according to formula (Fucα2)ₙGalβ4GlcNAc, wherein n is 0 or 1.

The amount of lectin used in a solution is about 0.1-500 µg/ml, preferably about 5-200 µg/ml or about 10-150 µg/ml. The amount of lectin for immobilization of the cell culture surface is about 0.001-50 µg/cm², preferably from about 0.01-50 µg/cm² to about 0.1-30 µg/cm², more preferably about 0.3-10 µg/cm² for a lectin with Mw of about 50 kDa, or corresponding molar density per surface area used. In one embodiment, about 1-50 µg/cm², or about 5-40 µg/cm², preferably about 10-40 µg/cm² of lectin is used in a solution to coat a plastic cell culture surface. In one embodiment, the concentration in the coating solution is between about 50-200 µg/ml for a lectin with Mw of about 50 kDa or corresponding molar density per surface area used. In a specific embodiment, a plastic cell culture well with polystyre surface is coated by passive adsorbtion using about 140 µg/ml solution in amount of about 30 µg/cm² for a lectin with Mw of about 50 kDa.

The present specification relates to a method for culturing human embryonic stem cells (hESC) and/or induced pluripotent stem (iPS) cells or a hESC polulation and/or a iPS cell population with a lectin. The specification is also directed to a culture medium composition comprising a lectin as a matrix. Further, the specification is directed to the use of a lectin in a method for culturing hESCs and/or iPS cells.

In one embodiment, the specification is directed to a method for culturing hESCs and/or iPS cells with at least one lectin and to a culture medium composition comprising at least one lectin. Further, the specification is directed to the use of at least one lectin in a method for culturing hESCs and/or iPS cells.

In one embodiment of the specification, the method for culturing HECSs and/or iPS cells refers to a method for maintenance of the undifferentiated state of the cells i.e., a method that promotes the growth of the cells but does not induce the differentiation of the cells.

In one embodiment of the specification the lectin is a natural plant lectin such as ECA lectin and in another embodiment of the specification at least one of the lectins is ECA lectin.

In one embodiment, the specification is directed to method for culturing hESCs and/or iPS cells with a lectin as a culturing matrix and a definitive, serum- and feeder-free medium. The specification is also directed to a culture medium composition comprising a lectin and a definitive, serum- and feeder-free medium. Further, the specification is directed to the use of a lectin together with a definitive, serum- and feeder-free media in a method for culturing hESCs and/or iPS cells.

In another embodiment, the specification is directed to method for culturing hESCs and/or iPS cells with at least one lectin and a definitive, serum- and feeder-free medium and to a culture medium composition comprising at least one lectin and a definitive, serum- and feeder-free medium. Further, the specification is directed to the use of at least one lectin together with a definitive, serum- and feeder-free media in a method for culturing hESCs and/or iPS cells.

The method for culturing hESCs and/or iPS cells according to the present specification comprises forming a cell culture surface and/or matrix containing a lectin, inoculating an/or transferring the cells to the surface containing the lectin and culturing the cells up to the desired number of passages in the surface or matrix. The method for culturing hESCs and/or iPS cells according to the present specification comprises optionally a step for addition of a definitive or fully-defined, serum- and feeder-free medium into the culture system. The method may also contain additional and/or optional steps that are conventional to methods of culturing cells, such as washing, incubating and divinding the cell populations.

In one embodiment of the specification, the method for culturing hESCs and/or iPS cells comprises coating of cell culture plates or vessels with a lectin, inoculating an/or transferring the cells onto the lectin coated plates or vessels and culturing the cells up to the desired number of passages. In another embodiment of the specification, the method for culturing hESCs and/or iPS cells comprises coating of cell culture plates or vessels with a lectin, inoculating an/or transferring the cells onto the lectin coated plates or vessels, adding a definitive or fully-defined, serum- and feeder-free medium into the culture system and culturing the cells up to the desired number of passages.

A definitive or fully-defined, serum- and feeder-free medium is a medium that is specifically formulated for the uniform growth of hESCs and/or ESC-like cells, such as iPS cells and contains ingredients required for maintaining normal morphology, pluripotency and differentation capability of hESCs and/or ESC-like cells, such as iPS cells. A definitive or fully-defined, serum- and feeder-free medium is free or essentially free of animal based and/or animal derived i.e., non-human ingredients. The serum- and feeder-free medium contains typically essential and non-essential amino acids, vitamins, growth factors, inorganic salts, trace elements and other components such as sugars, fatty acids and antibiotics. The relative amount of a specific ingredient depends on the quality and quantity of the other ingredients selected to the medium composition and on the manufacturer of the medium, for example. StemPro^{®} hESC SFM, developed and sold by Invitrogen Corporation, US, and mTESR™, developed and sold by Stem Cell Technologies, US, are examples of this kind of a definitive, serum- and feeder-free medium developed for culturing of hESCs without feeder cells, but there are also many others with similar properties.

In a further embodiment of the specification, the definitive, serum- and feeder-free medium is StemPro^{®} hESC SFM.

According to the present specification, a lectin is used as a sole culture matrix ingredient or it is added to a culture media applicable to the growth of hESCs and/or iPS cells or used with such a medium. The culture media can also be supplemented, for example, with a single or a plurality of growth factors selected from, for example, a WNT signaling agonist, TGF-b, bFGF, IL-6, SCF, BMP-2, thrombopoietin, EPO, IGF-1, IL-11, IL-5, Flt-3/Flk-2 ligand, fibronectin, LIF, HGF, NFG, angiopoietin-like 2 and 3, G-CSF, GM-CSF, Tpo, Shh, Wnt-3a, Kirre, or a mixture thereof.

In one embodiment of the specification, the hESCs and/or iPS cells are grown on a lectin, such as a plant lectin or galectin coated plate or vessel.

The hESCs and/or iPS cells cultured according to the present specification are not exposed to animal-derived material during their cultivation, at least not in such an extent than cells cultured according to the known methods using feeder cells, Matrigel™ and/or other animal-derived material.

The hESCs cultured according to the present specification have shown to have the typical characteristics of human embryonal stem cells, posing telomerase activity and expressing surface markers SSEA-3, Tra-1-60 and Tra-1-81. In addition, the cells have been shown to be able to differentiate by forming embryoid bodies and/or teratomas.

The iPS cells cultured according to the present specification have shown to express surface markers SSEA-3 and Tra-1-60 as well as express Oct-4 and Nanog genes, that are characteristics to pluripotent cells such as embryonal stem cells. They also were able to form teratomas, an essential indicator for pluripotency.

The method and the culture medium composition of the present specification provide means for culturing hESCs and/or iPS cells substantially free of xenogenic contamination. The hESCs, iPS cells and/or cell population(s) cultured according to the present specification are thus safe for the current and future transplantation applications.

The following examples represent illustrative embodiments of the specification without limiting the invention as defined in the claims in any way.

### EXAMPLE 1 (reference example)

### Human embryonic stem cell (hESC) lines cultured on ECA -lectin coated plastic

Processes for generation of hESC lines from blastocyst stage of *in vitro* fertilized human embryos have been described previously in Thomson et al. (Science, 282:1145-1147, 1998). Cell lines FES 22, FES 29 and FES 30 were initially derived and cultured either on mouse embryonic fibroblasts feeders (MEFs; 12-13 pc fetuses of the ICR strain), or on human foreskin fibroblast feeder cells (HFFs; CRL-2429 ATCC, Mananas, USA) as disclosed in Mikkola et al. BMC Dev Biol, 6:40-51, 2006. All the lines were cultured in serum-free medium (KnockOut™ D-MEM; Gibco® Cell culture systems, Invitrogen, Paisley, UK) supplemented with 2mM L-Glutamin/Penicillin streptomycin (Sigma-Aldrich), 20% KnockOut Serum Replacement (Gibco), 1 X non-essential amino acids (Gibco), 0.1 mM β-mercaptoethanol (Gibco), 1 X ITS (Sigma-Aldrich) and 4 ng/ml bFGF (Sigma/Invitrogen) on feeder cells, or on Matrigel™ (BD Biosciences) in the same medium (supplemented with additional 4 ng/ml bFGF) conditioned over night on MEFs. Passaging was done either mechanically or enzymatically using collagenase IV (Gibco).

### ECA-lectin coating of cell culture plates

ECA-lectin (EY laboratories, USA) was dissolved in phosphate buffered saline 140µg/ml. Lectin dilution was sterile filtrated using Millex-GV syringe driven filter units (0.22 µm, SLGV 013 SL, Millipore, Ireland) and allowed to passively adsorb on cell culture plate by overnight incubation at +4°C. After incubation the wells were washed three times with phosphate buffered saline and stem cells were plated on them.

### hESC culturing on ECA-lectin coated cell culture plates

The hESC lines (FES 22, FES 29, FES 30) were cultured at least three passages on Matrigel™ before transferring them onto ECA coated plates, FES 29 was transferred also directly from MEFs onto ECA coated plates in conditioned medium. All lines were maintained on Matrigel™ as controls. The growing cell aggregates were then passaged to new plates at 3-7 day intervals.

### hESC embryoid body (EB) formation

EBs were generated as previously described in Mikkola et al. (2006) with small modifications. Briefly, to induce the formation of EBs the confluent hESC colonies were first treated with 200 U/ml collagenase IV and transferred on non-adherent Petri dishes to form suspension cultures. The formed EBs were cultured in suspension for the next 10 days in standard culture medium (see above) without bFGF.

### Teratoma assay

In order to study teratoma formation about 200 000 morphologically good looking hESCs were injected into the testes of nude mice. The resulting tumors were harvested 8 weeks later and fixed with formalin for immunohistological examination as decribed in Mikkola et al. (2006).

### Flow Cytometry

hESCs were detached enzymatically and washed in 1% ultra pure BSA in PBS. Monoclonal antibodies against SSEA-3, Tra-1-60 and Tra-1-81 (1:50; gifts kindly provided by ESTOOLS www.estools.org) were used as markers for undifferentiated hESCs. Staining was performed according to manufacturer's instructions. FACS analysis was done with FACS Calibur machinery and CellquestPro software (Becton Dickinson).

### Results

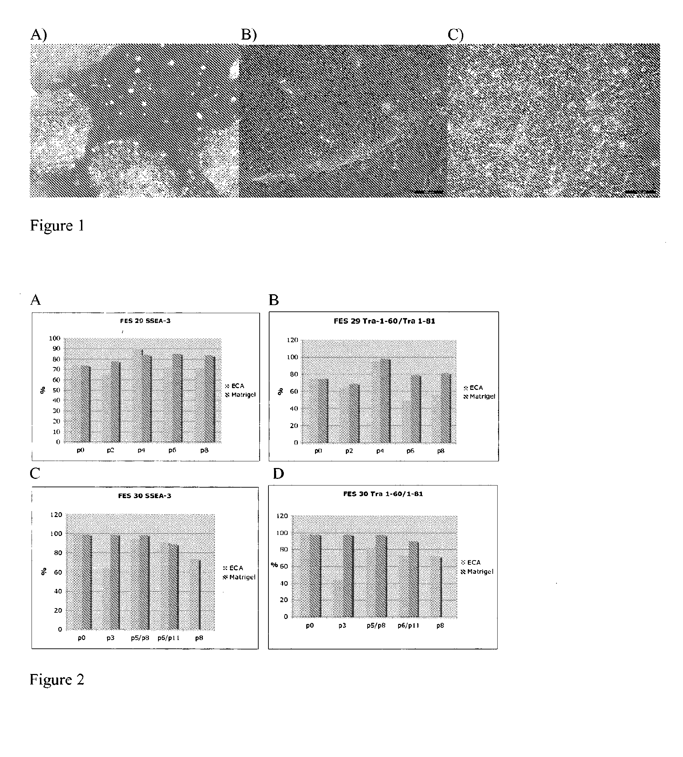

Three different hESC-lines, FES 22, FES 29 and FES 30, were cultured on ECA-coated wells in MEF-conditioned medium up to 23 passages. The morphology of hESCs was similar to the control Matrigel cultures and hESCs looked undifferentiated after ECA-lectin passages (Figure 1). Lines FES 29 and FES 30 were repeatedly successfully transferred from Matrigel to ECA-plates. FES 29 cells were also transferred onto ECA-lectin straight from feeder cells (MEFs).

The expression of surface markers of undifferentiated hESCs (SSEA-3 and Tra-1-60/Tra-1-81) were analyzed every 2 or 3 passages by flow cytometry. The follow-up of the surface marker expression during the culture of FES 29 and FES 30 cells on ECA is shown in Figure 2.

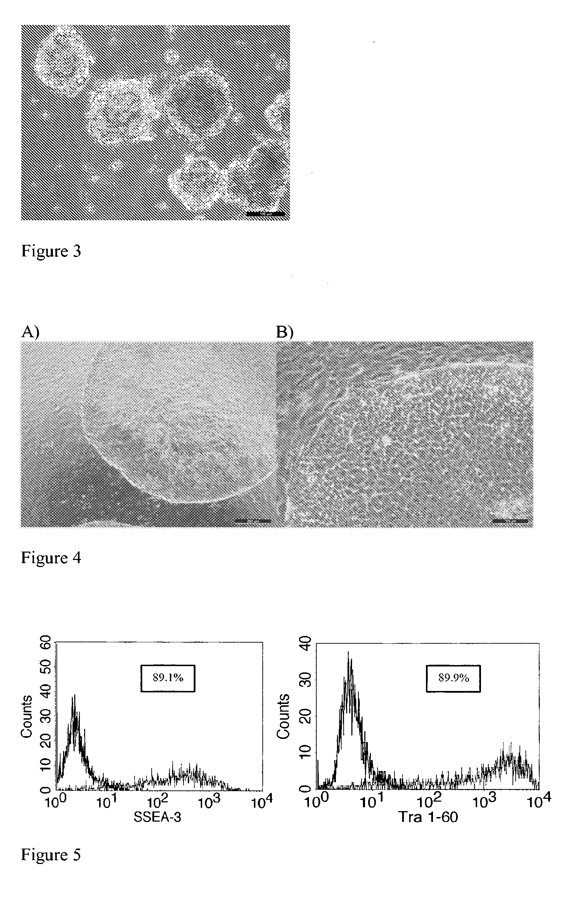

The pluripotency of hESCs after several ECA passages was verified by their ability to form EBs in suspension culture or teratomas in nude mice. FES 30 cells cultured 23 passages on ECA and FES 29 cells cultured 4 passages on ECA formed teratoma-containing tissues from all three germ cell layers (data not shown). EBs were successfully formed from FES 29 and FES 30 cells after ECA-culture (Figure 3).

### EXAMPLE 2 (reference example)

### Culturing hESCs on ECA lectin in definitive medium

### Culturing hESCs

The FES 29 hESC line (see example 1) was cultured 14 passages on Matrigel™ before transferring the cells on ECA-lectin coated plates. The hESCs were cultured on ECA-lectin coated plates for 7 passages in MEF-conditioned medium and then changed to a definitive medium, StemPro^{®} hESC SFM (Gibco, Invitrogen A10007-01, 2007/2008). Matrigel™ was used as a control. For enzymatic passaging the cells were exposed to 200 units/ml collagenase IV (Gibco) for 1-2 min at 37°C, washed once in PBS and dissociated by gently pipetting and plated on 2-3 new dishes.

### hESC embryoid body (EB) formation

EBs were generated as previously described in Mikkola et al. (2006). Briefly, to induce the formation of EBs the confluent hESC colonies were first treated with 200 U/ml collagenase IV and then transferred on non-adherent Petri dishes to form suspension cultures. The formed EBs were cultured in suspension for the next 10 days in the standard culture medium (see above) without bFGF.

### Flow cytometry

hESCs were detached enzymatically and washed with 1% ultra pure BSA in PBS. Monoclonal antibodies against SSEA-3, Tra-1-60 and Tra-1-81 (1:50; gifts kindly provided by ESTOOLS www.estools.org) were used as markers for undifferentiated hESCs. Staining was performed according to manufacturer's instructions. FACS analysis was done with FACS Calibur machinery and CellQuestPro software (Becton Dickinson).

### Results

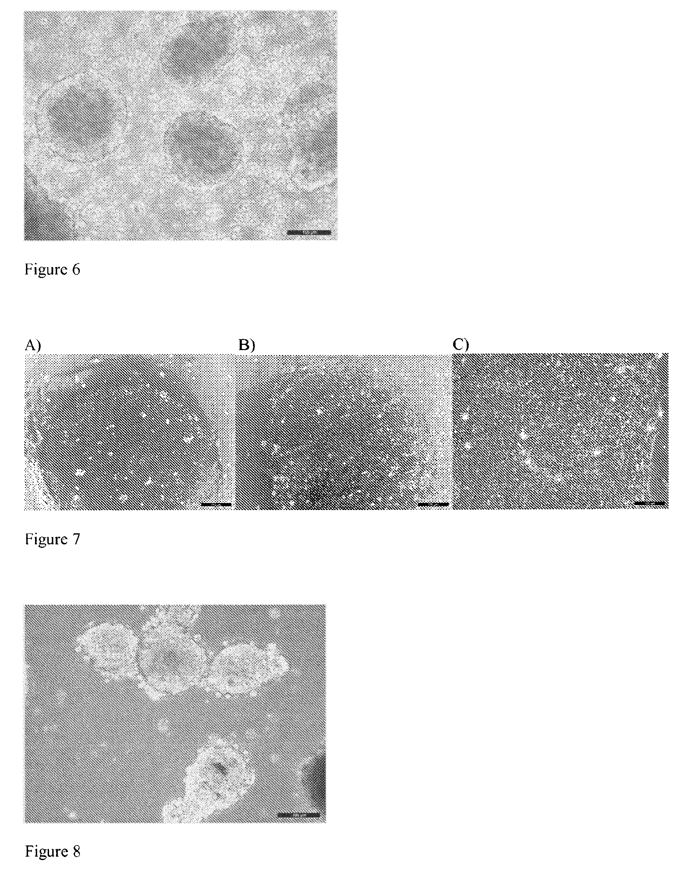

hESCs, FES 29 line, were cultured on ECA-lectin for 7 passages with MEF-conditioned culture medium. In the 8^{th} passage conditioned medium was changed to the commercial definitive medium, StemPro^{®} hESC SFM. FES 29 cells maintained their undifferentiated state and pluripotency during up to 5 passages in definitive medium on ECA. In Figure 4 the typical hESC colonies on ECA in the StemPro^{®} medium are shown. FACS-analysis of the expression of surface markers of undifferentiated hESCs (SSEA-3 and Tra-1-60) is presented in Figure 5. EBs were formed after 12 passages on ECA and 4 passages in the StemPro^{®} medium (Figure 6).

### EXAMPLE 3

### Culturing induced pluripotent stem (iPS) cells on ECA lectin coated plastic Culturing iPS cells

Two lines of iPS cells were originated either from human embryonal lung fibroblasts or human (child under 18 years) foreskin fibroblasts with protocol modified from Okita et al. (Nature, 448:313-317, 2007) and Wernig et al. (Nature, 448:318-324, 2007). FiPS1-5 and FiPS6-12 lines were cultured for 10 or 8 passages on MEFs before transferring them onto ECA or Matrigel™ coated dishes in MEF-conditioned medium. For enzymatic passaging the cells were subjected to 200 units/ml collagenase IV for 1-2 min at 37°C, washed once in PBS and dissociated by gently pipetting and plated on 2-3 new dishes.

### hESC embryoid body (EB) formation

EBs were generated as previously described in Mikkola et al. (2006). Briefly, to induce the formation of EBs the confluent cell colonies were first treated with 200 U/ml collagenase IV and then transferred on non-adherent Petri dishes to form suspension cultures. The formed EBs were cultured in suspension for the next 10 days in standard culture medium (see above) without bFGF.

### Results

iPS-cells were cultured in similar *in vitro* conditions as hESCs. Two iPS cell-lines, FiPS1-5 and FiPS6-12, were transferred from MEFs (after passage 10 or 8, respectively) to Matrigel or to ECA-coated plates in MEF-conditioned medium. iPS cells were morphologically similar to hESCs in all culturing conditions (Figure 7). EBs were formed from FiPS6-12 cells after 6 passages on ECA (Figure 8).

### EXAMPLE 4

### Culture of induced pluripotent stem (iPS) cell line FiPS 6-14 on ECA

The iPS cell line was originated from human fibroblasts essentially as described by Takahashi et al (Cell 131:861-872), Okita et al. (Nature, 448:313-317, 2007) and Wernig et al. (Nature, 448:318-324, 2007). FiPS6-14 line was cultured on MEFs before transferring onto ECA or Matrigel™ coated dishes in MEF-conditioned medium. For enzymatic passaging the cells were subjected to 200 units/ml collagenase IV for 1-2 min at 37°C, washed twice in DMEM/F12 and dissociated by gently pipetting and plated on 2-3 new dishes.

Two parallel cultures, called here FiPS 6-14#p18 and FiPS 6-14#p21, were set up. The two cell cultures were cultured for 13 and 12 passages, respectively, on ECA in MEF-conditioned medium.

Cell surface expression of Tra-1-60 and SSEA-3 stem cell markers were analysed by flow cytometry. Briefly, the cells were detached enzymatically and washed in 1% ultra pure BSA in PBS- 2mM EDTA supplemented with 0,1% NaN₃. Monoclonal antibodies against SSEA-3 (1:20; provided by ESTOOLS www.estools.org) and Tra-1-60 (1:20; TRA-1-60, Chemicon, cat. n:o MAB4360) were used. Secondary antibodies PE mouse anti-rat IgM (BD Pharmingen) and FITC rabbit anti-mouse IgM (Jackson ImmunoResearch) were used at 1:50 dilution and staining was performed according to manufacturer's instructions. Fluorescence-activated cell sorting (FACS) analysis was done with FACSAria apparatus and FACSDiva software (Becton Dickinson).

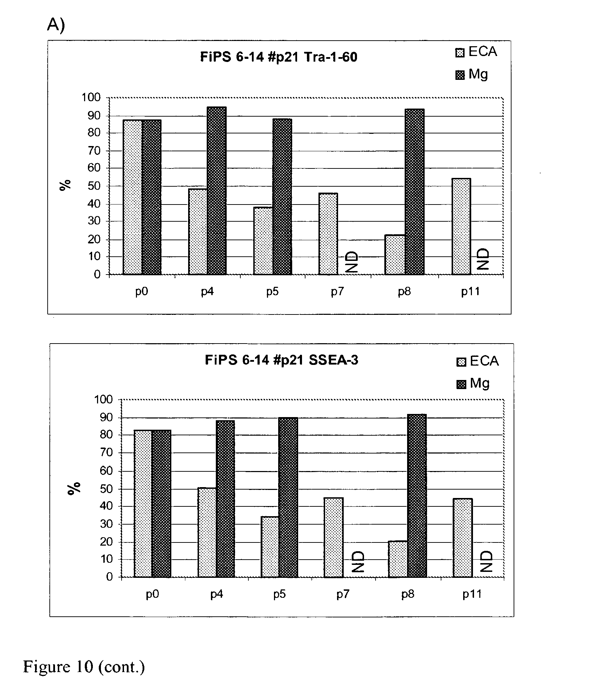

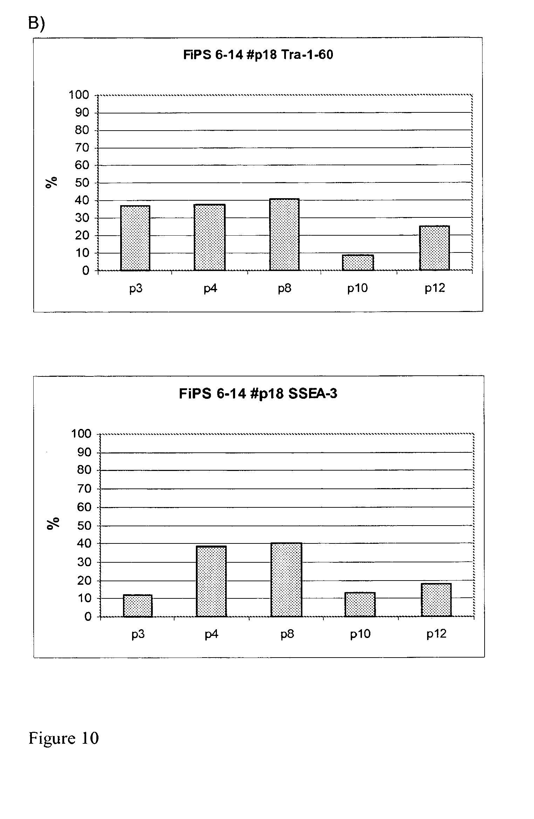

In Figure 10 surface expression of two markers for stem cellness, Tra-1-60 and SSEA-3, on the FiPS 6-14 cells as determined by standard FACS analysis is shown. The results confirmed that the two parallel iPS cultures, FiPS 6-14#p21 and FiPS 6-14#p18, were positive for both ESC surface markers at least up to 11 and 12 passages, respectively. The average expression levels on the ECA surface were, however, about 30-40% of those determined for the cells cultured on Matrigel™ matrix (FiPS 6-14#p21, Figure 10A).

The ability of ECA to support the growth of iPS cells in conditioned medium was tested using FiPS 6-14#p21 and StemPro™ medium. The cells were cultivated for 2 passages. They were morphologically normal as determined by CellIQ imaging and they expressed the stem cell markers as determined by FACS analysis: Tra-1-60 was found in 55,8% of the FiPS cells (control cells cultivated on MEF conditioned medium showed 54.2% staining) and SSEA-3 in 79.5% (control cells 44.5%).

Total mRNa was extracted from FiPS-6-14 cells using Nucleospin RNA II (Macherey Nagel) Rna extraction kit. Contaminating nuclear DNA was digested by rDNase (included by KIT) to remove DNA template.

Reverse transcription of RNA was carried out using High-Capacity cDNA reverse transcription kit (Applied Biosystems) according to manufactures protocol.

Quantitative PCR was carried out by using gene specific probes and primers purchased from Applied Biosystems. Taqman Gene expression assays: Nanog Hs02387400_g1, Tata Box binding protein Hs99999910_m1, Oct-4 Hs01895061_u1. Samples were run in duplicates or triplicates, containing 30ng cDNA (calculated from RNA measurement) each. qPCR reactions were run by Abi Prism 7000 Sequence Detection systems, results analysed by sequence detection software, vesion 1.2.3. (Applied Biosystems).

In Figure 11 mRNA expression levels of Oct-4 (both FiPS 6-14#p18 and FiPS 6-14#p21) and Nanog (FiPS 6-14#p21 only), both established mRNA markers for stem cellness, are shown. The analysis confirmed that all the iPS cell samples up to 10 passages tested were positive for the two undifferentiated ESC marker genes. Furthermore, the iPS cells cultured up to 7 passages on ECA lectin express both marker genes at levels comparable to or even slightly higher than those observed for the cells cultured on the control Matrigel™ matrix (FiPS 6-14#p21, Figure 10B). The expression profiles of the marker genes were very similar throughout the passages (FiPS 6-14#p21, Figure 11B).

Karyotype of cell line FiPS 6-14#p18 was determined before and after culturing 13 passages on ECA and was found to be normal. The standard karyotyping was done by Medix, Ltd (Helsinki, Finland).

### EXAMPLE 5

### Culture of induced pluripotent stem (iPS) cell line FiPS 5-7 on ECA

The iPS cell line was originated from human fibroblasts essentially as described by Takahashi et al (Cell 131:861-872), Okita et al. (Nature, 448:313-317, 2007) and Wernig et al. (Nature, 448:318-324, 2007). FiPS6-14 line was cultured on MEFs before transferring onto ECA or Matrigel™ coated dishes in MEF-conditioned medium. For enzymatic passaging the cells were subjected to 200 units/ml collagenase IV for 1-2 min at 37°C, washed twice in DMEM/F12 and dissociated by gently pipetting and plated on 2-3 new dishes.

The cell line was cultivated on ECA in MEF-conditioned medium up to 27 passages. mRNA expression levels of Oct-4 and Nanog were determined to verify that the cells remained ESC-like cells.

Total mRNa was extracted from FiPS 5-7 cells using Nucleospin RNA II (Macherey Nagel) Rna extraction kit. Contaminating nuclear DNA was digested by rDNase (included by KIT) to remove DNA template.

Reverse transcription of RNA was carried out using High-Capacity cDNA reverse transcription kit (Applied Biosystems) according to manufactures protocol.

Quantitative PCR was carried out by using gene specific probes and primers purchased from Applied Biosystems. Taqman Gene expression assays: Nanog Hs02387400_g1, Tata Box binding protein Hs99999910_m1, Oct-4 Hs01895061_u1. Samples were run in duplicates or triplicates, containing 30ng cDNA (calculated from RNA measurement) each. qPCR reactions were run by Abi Prism 7000 Sequence Detection systems, results analysed by sequence detection software, vesion 1.2.3. (Applied Biosystems).

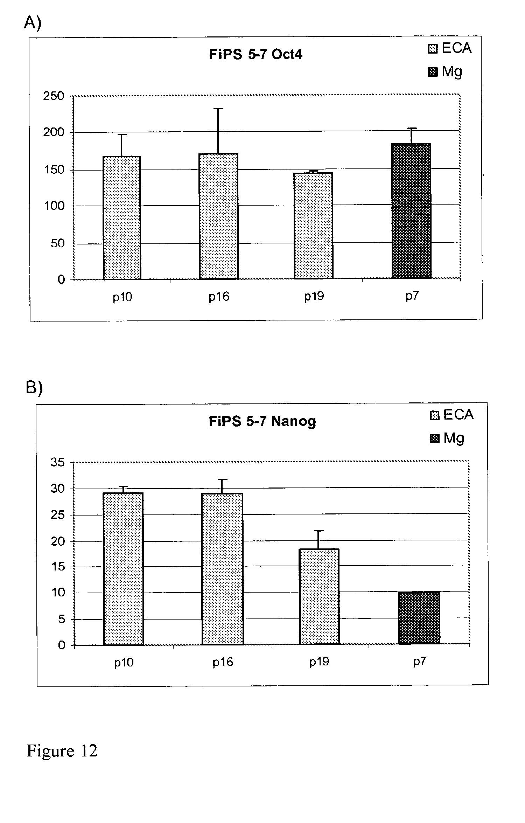

In Figure 12 mRNA expression levels of stem cell markers Oct-4 (Figure 12A) and Nanog (Figure 12B) are shown. The stem cell marker expression from FiPS 5-7 cultured on ECA was analysed at passages 10, 16 and 19. After 10 passages on ECA, the cells were cultured on Matrigel for 7 further passages and the stem cell marker expression was analysed (p7 Mg in Figures 12A and 12B). The FiPS 5-7 cells cultured even up to 19 passages on ECA lectin expressed evidently both marker genes Oct-4 and Nanog. The expression levels of Oct-4 in FiPS 5-7 cells cultured the first 10 passages on ECA followed by parallel cultivation for 6 passages on ECA and for 7 passages on the control Matrigel™ matrix were comparable as seen in Figure 12A.

Karyotype of cell line FiPS 5-7 was determined after culturing 12 passages on ECA and was found to be normal. The standard karyotyping was done by Medix, Ltd (Helsinki, Finland).

### EXAMPLE 6 (reference example)

### Human embryonic stem cell (hESC) lines cultured on ECA lectin coated plastic maintain pluripotent stage

### Materials and Methods

Process for generation of hESC lines from blastocyst stage of *in vitro* fertilized human embryos have been described previously in Thomson et al. (Science, 282:1145-1147, 1998). The cell line FES 29 was initially derived and cultured on human foreskin fibroblast feeder cells (HFFs; CRL-2429 ATCC, Mananas, USA) as described in Mikkola et al. (2006). FES 29 was cultured either in commercial defined medium StemPro® hESC SFM (InVitrogen, Paisley, UK) or in conditioned serum-free medium (CM-medium): KnockOut™ D-MEM (Gibco® Cell culture systems, Invitrogen, Paisley, UK) supplemented with 2 mM L-Glutamin/Penicillin streptomycin (Sigma-Aldrich), 20% KnockOut Serum Replacement (Gibco), 1 X non-essential amino acids (Gibco), 0.1 mM β-mercaptoethanol (Gibco), 1 X ITS (Sigma-Aldrich) and 8 ng/ml bFGF (Sigma/Invitrogen) conditioned over night on mouse fibroblast feeder cells. Cells were cultured either on Matrigel™ (cat n:o 356231 BD Biosciences) as a control culture or on ECA lectin (Ey Laboratories, tested product). Passaging was done enzymatically using collagenase IV (Gibco cat n:o 17104-019).

### ECA-coating of cell culture plates

ECA lectin (EY laboratories, USA, Cat n:o L5901) was dissolved in phosphate buffered saline (PBS) 140 µg/ml. Lectin dilution was sterile filtrated using Millex-GV syringe driven filter units (0.22 µm, SLGV 013 SL, Millipore, Ireland) and allowed to passively adsorb on cell culture plate by overnight incubation at +4°C. After incubation the wells were washed three times with PBS and stem cells were plated on them.

### Quantitative RT-PCR (qRT-PCR) analysis

For qRT-PCR analysis FES 29 cell samples were harvested in lysis buffer included in RNA extraction kit (NucleoSpin RNA II, Macherey-Nagel, Düren, Germany) and stored at -70°C. Extracted RNA was purified (NucleoSpin RNA Cleanup-kit, Macherey-Nagel) and concentration of RNA was measured by spectrophotometer. Reverse transcription (RT) was performed using M-MLV-RTase (Promega) in final concentration of 5 U/µl for 90 minutes at +37°C and RT enzyme was inactivated at +95°C for 5 minutes. Quantitative PCR was performed with SYBR Green I (Molecular Probes/In Vitrogen) and Roche Applied Biosystem reagents (enzyme: AmpliTaq Cold™). Cyclophilin G was used as an endogenous control. qPCR was performed with CAS-1200 pipetting robot and Corbett PCR machinery in 20µl reaction volume.

### Teratoma assay

About 200000 morphologically good looking hESCs were injected into nude mice testis. The resulting tumors were harvested 8 weeks later and fixed with formalin for immunohistological examination as described in Mikkola et al. (2006).

### Results

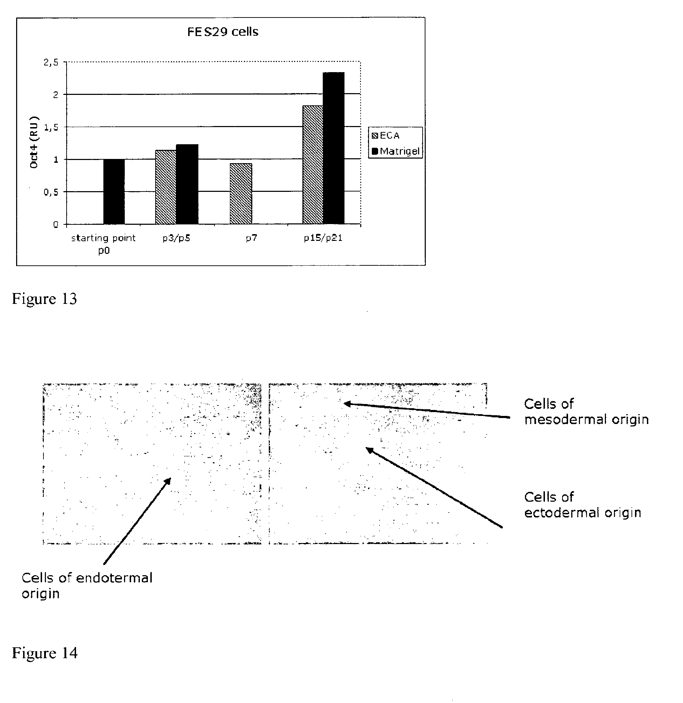

Quantitative RT-PCR analysis and teratoma assay have been used to demonstrate pluripotency of ECA-cultured FES 29 hESCs. For qRT-PCR the cells were cultured on ECA lectin (up to 15 passages) and on Matrigel™ (up to 21 passages) in the CM-medium and cell samples were collected for quantitative RT-PCR (qRT-PCR) every 2 or 3 passages. A selected part of the samples were analysed by qRT-PCR to determine the mRNA expression levels of Oct-4 which was used as the marker of undifferentiated and pluripotent hESCs. The mRNA expression of Oct-4 remained in comparable levels when culturing cells on ECA lectin compared to those cultured on Matrigel™ (Figure 13). The expression of Oct-4 mRNA doubled in late passages but in the same way on both ECA and Matrigel™ matrices. These results suggest that hESCs cultured on ECA lectin remain in an undifferented stage.

Pluripotency of the hESCs *in vivo* was assessed by the ability of the FES 29 cells to form teratomas in mice. For this, the hESCs were cultured for totally 15 passages on ECA lectin and the last 8 passages in StemPro medium before transplantation of the FES 29 cells into an immunodeficient mouse. The results of the teratoma assay (Figure 14) confirmed that the hESCs cultivated on ECA lectin surface in defined medium kept their differentiation ability as demonstrated by formation of the three germ layer derivatives (endoterm, mesoderm and ectoderm).

## Claims

1. A method for culturing human induced pluripotent stem (iPS) cells, **characterized in that** said cells are contacted with at least one lectin as a matrix or attached on a culturing plate, and the lectin recognizes the structure (Fucα₂)ₙGalβ4GlcNAc, wherein n is 0 or 1.

2. The method of claim 1, **characterized in that** said cells are not exposed to animal-derived material during their cultivation.

3. The method of claim 1 **characterized in that** said cells are cultured with the lectin as a matrix and in a fully-defined, serum- and feeder-free medium.

4. The method of claim 1, **characterized in that** the method comprises forming a cell culture surface or a matrix containing the lectin, inoculating and/or transferring the cells to the surface containing the lectin and culturing the cells up to the desired number of passages in the surface or matrix.

5. The method of claim 1 **characterized in that** the method comprises coating of cell culture plates or vessels with a lectin, inoculating and/or transferring the cells onto the lectin coated plates or vessels, adding a fully-defined, serum- and feeder-free medium into the culture system and culturing the cells up to the desired number of passages.

6. The method of claim 1, **characterized in that** said cells are maintained in undifferentiated state.

7. The method of claim 1, **characterized in that** the lectin is ECA, UEA-1, DSA or galectin.

8. A culture medium composition for culturing human induced pluripotent stem (iPS) cells, **characterized in that** it comprises at least one lectin as a matrix, wherein said lectin recognizes the structure (Fucα2)ₙGalβ4GlcNAc, wherein n is 0 or 1, and induced pluripotent stem (iPS) cells.

9. The composition of claim 8, **characterized in that** the medium is a fully-defined, serum- and feeder-free medium.

10. The composition of claim 8, **characterized in that** the lectin is ECA, UEA-1, DSA or galectin.

11. Use of one or more lectins in a method for culturing human iPS cells, wherein said one or more lectin recognize(s) the structure (Fucα2)ₙGalβ4GlcNAc, wherein n is 0 or 1, and wherein one or more lectins is used as a matrix together with a fully-defined, serum- and feeder-free medium.

12. The use of claim 11, wherein the lectin is used as a sole culture matrix ingredient.

13. The use of claim 11, wherein at least one of the lectins is ECA, UEA-1, DSA or galectin.

## Patentansprüche

1. Verfahren zum Züchten humaner induzierter pluripotenter Stammzellen (iPS-Zellen), **dadurch gekennzeichnet, dass** die Zellen mit mindestens einem Lectin als Matrix in Kontakt gebracht oder auf eine Kulturplatte aufgebracht werden und das Lectin die Struktur (Fucα2)ₙGalβ4GlcNAc erkennt, wobei n 0 oder 1 ist.

2. Verfahren nach Anspruch 1, **dadurch gekennzeichnet, dass** die Zellen während ihrer Züchtung keinem Material ausgesetzt werden, das von einem Tier stammt.

3. Verfahren nach Anspruch 1, **dadurch gekennzeichnet, dass** die Zellen mit dem Lectin als Matrix und in einem vollständig definierten, Serum- und Feeder-freien Medium gezüchtet werden.

4. Verfahren nach Anspruch 1, **dadurch gekennzeichnet, dass** das Verfahren das Bilden einer Zellkulturoberfläche oder einer Matrix, die das Lectin enthält, das Beimpfen der Oberfläche mit Zellen und/oder das Transferieren der Zellen auf die Oberfläche, die das Lectin enthält, und das Züchten der Zellen bis zur gewünschten Anzahl an Passagen in der Oberfläche oder Matrix umfasst.

5. Verfahren nach Anspruch 1, **dadurch gekennzeichnet, dass** das Verfahren das Beschichten der Zellkulturplatten oder -gefäße mit einem Lectin, das Beimpfen der Lectin-beschichteten Platten oder Gefäße mit Zellen und/oder das Transferieren der Zellen auf die Lectin-beschichteten Platten oder Gefäße, das Hinzufügen eines vollständig definierten, Serum- und Feeder-freien Mediums zum Kultursystem und Züchten der Zellen bis zur gewünschten Anzahl an Passagen umfasst.

6. Verfahren nach Anspruch 1, **dadurch gekennzeichnet, dass** die Zellen in undifferenziertem Zustand gehalten werden.

7. Verfahren nach Anspruch 1, **dadurch gekennzeichnet, dass** das Lectin ECA, UEA-1, DSA oder Galectin ist.

8. Kulturmediumzusammensetzung zum Züchten humaner induzierter pluripotenter Stammzellen (iPS-Zellen), **dadurch gekennzeichnet, dass** es mindestens ein Lectin als Matrix, wobei das Lectin die Struktur (Fucα2)ₙGalβ4GlcNAc erkennt, wobei n 0 oder 1 ist, und induzierte pluripotente Stammzellen (iPS-Zellen) umfasst.

9. Zusammensetzung nach Anspruch 8, **dadurch gekennzeichnet, dass** das Medium ein vollständig definiertes, Serum- und Feeder-freies Medium ist.

10. Zusammensetzung nach Anspruch 8, **dadurch gekennzeichnet, dass** das Lectin ECA, UEA-1, DSA oder Galectin ist.

11. Verwendung eines Lectins oder mehrerer Lectine in einem Verfahren zum Züchten humaner iPS-Zellen, wobei das eine Lectin oder die mehreren Lectine die Struktur (Fucα2)ₙGalβ4GlcNAc erkennt/erkennen, wobei n 0 oder 1 ist, und wobei das eine Lectin oder die mehreren Lectine zusammen mit einem vollständig definierten, Serum- und Feeder-freien Medium als Matrix verwendet wird/werden.

12. Verwendung nach Anspruch 11, wobei das Lectin als einziger Kulturmatrixbestandteil verwendet wird.

13. Verwendung nach Anspruch 11, wobei mindestens eines der Lectine ECA, UEA-1, DSA oder Galectin ist.

## Revendications

1. Procédé de culture de cellules souches pluripotentes induites (iPS) humaines, **caractérisé en ce que** lesdites cellules sont mises en contact avec au moins une lectine en tant que matrice ou fixée sur une plaque de culture, et la lectine reconnaît la structure (Fucα2)ₙGalβ4GlcNAc, dans laquelle n vaut 0 ou 1.

2. Procédé selon la revendication 1, **caractérisé en ce que** lesdites cellules ne sont exposées à aucun matériau d'origine animale durant leur culture.

3. Procédé selon la revendication 1, **caractérisé en ce que** lesdites cellules sont cultivées avec la lectine en tant que matrice et dans un milieu totalement défini, dépourvu de sérum et de cellule nourricière.

4. Procédé selon la revendication 1, **caractérisé en ce que** le procédé comprend la formation d'une surface de culture cellulaire ou d'une matrice contenant la lectine, l'inoculation et/ou le transfert des cellules sur la surface contenant la lectine et la culture des cellules jusqu'au nombre souhaité de repiquages dans la surface ou la matrice.

5. Procédé selon la revendication 1, **caractérisé en ce que** le procédé comprend le revêtement de plaques ou récipients de culture cellulaire avec une lectine, l'inoculation et/ou le transfert des cellules sur des plaques ou des récipients revêtus de lectine, l'ajout d'un milieu totalement défini, dépourvu de sérum et de cellule nourricière, dans le système de culture et la culture des cellules jusqu'au nombre souhaité de repiquages.

6. Procédé selon la revendication 1, **caractérisé en ce que** lesdites cellules sont maintenues dans un état non différencié.

7. Procédé selon la revendication 1, **caractérisé en ce que** la lectine est l'ECA, l'UEA-1, la DSA ou la galectine.

8. Composition de milieu de culture pour la culture de cellules souches pluripotentes induites (iPS) humaines, **caractérisée en ce qu'**elle comprend au moins une lectine en tant que matrice, dans laquelle ladite lectine reconnaît la structure (Fucα2)ₙGalβ4GlcNAc, dans laquelle n vaut 0 ou 1, et des cellules souches pluripotentes induites (iPS).

9. Composition selon la revendication 8, **caractérisée en ce que** le milieu est un milieu totalement défini, dépourvu de sérum et de cellule nourricière.

10. Composition selon la revendication 8, **caractérisée en ce que** la lectine est l'ECA, l'UEA-1, la DSA ou la galectine.

11. Utilisation d'une ou plusieurs lectines dans un procédé de culture de cellules iPS humaines, dans laquelle ladite ou lesdites lectines reconnaissent la structure (Fucα2)ₙGalβ4GlcNAc, dans laquelle n vaut 0 ou 1, et dans laquelle une ou plusieurs lectines sont utilisées en tant que matrice conjointement à un milieu totalement défini, dépourvu de sérum et de cellule nourricière.

12. Utilisation selon la revendication 11, dans laquelle la lectine est utilisée en tant qu'unique ingrédient de matrice de culture.

13. Utilisation selon la revendication 11, dans laquelle au moins l'une des lectines est l'ECA, l'UEA-1, la DSA ou la galectine.
